# EUROPEAN PATENT SPECIFICATION

(11) **EP 3 955 865 B1**
(45) Date of publication and mention of the grant of the patent: **15.11.2023**
(21) Application number: 20717689.2
(22) Date of filing: 15.04.2020
(51) Int. Cl.: A61F 9/00, B01L 3/02, B65D 77/04

(54) **STORAGE CONTAINER FOR AT LEAST ONE PIPETTE**
AUFBEWAHRUNGSBEHÄLTER FÜR MINDESTENS EINE PIPETTE
RÉCIPIENT DE CONSERVATION POUR AU MOINS UNE PIPETTE

(30) Priority: 15.04.2019 EP 19169277
(43) Date of publication of application: 23.02.2022
(73) Proprietor: Siambanis, Tim, 24576 Weddelbrook (DE)
(72) Inventor: Siambanis, Tim, 24576 Weddelbrook (DE)
(74) Representative: Müller Schupfner & Partner Patent- und Rechtsanwaltspartnerschaft mbB
(86) International application number: PCT/EP2020/060638
(87) International publication number: WO 2020/212456

(56) References cited:
- US-A- 1 700 781
- US-A- 2 180 248
- US-A1- 2008 283 530

## Description

The present invention relates to a storage container with at least one removable cover for pipettes containing a fluid, in particular a treatment fluid for eyes, wherein at least one pipette is attachable with one end to the at least one cover for storage in the storage container, as well as a set consisting of the storage container and at least one pipette.

### Background on the Invention

For treating eye diseases or eye irritations, medicines or other active ingredients are applied locally to the eye. Eye salves and eye drops that are introduced as sterile, aqueous, or respectively oily solutions or suspensions into the conjunctival sac or dripped onto the cornea are among the most conventional treatment fluids. The problem during use is to reliably apply the active ingredient to a small space, for example due to blinking, and in the provided amount in order to achieve the desired effect. In addition, there is the necessary hygiene for avoiding infections to consider. Dry eyes are also burdensome and therefore require treatment with a treatment fluid in the form of an eye-wetting agent that supports the tear film. In particular people with abnormally dry eyes who suffer from keratoconjunctivitis sicca (dry eye syndrome) use artificial tear replacement fluid, and generally use it regularly spread throughout the day.

Depending on the frequency of use, different containers are appropriate for the purpose of storing and dispensing treatment fluids such as eye drops. Single dose systems and multiple dose systems are known. Multiple dose systems are for example drop bottles with several millimeters of content. So-called single dose containers belong to the classic single dose systems. These are small, transparent or milky-cloudy pipettes made of plastic that contain the fluid as a sterile solution. The sealing cap that closes the pipette at the front end is broken off, or respectively twisted off shortly before initial use.

At the other end of the pipette, commonly there is a tab with which the pipette is grasped. The tab does not contain any fluid. Generally, the pipette possesses walls made of soft plastic so that the user can exert pressure on the dispensing chamber of the pipette using two fingers that causes the treatment fluid to be dispensed from the dispensing chamber, for example into an eye.

Generally, after the pipette is opened, the treatment fluid contained therein is only usable for a limited time in order to, inter alia, ensure ongoing sterility. Aqueous preparations in multiple dose systems are usually preserved with additives and may be used for a while after opening, such as 1 to 4 weeks, sometimes even significantly longer, depending on the manufacturer's information.

Adding preservatives to single dose systems is generally undesirable, for example due to potential side effects, or respectively unnecessary since germs cannot penetrate into the closed pipette. The pipette is generally discarded after a single use; consequently, this use is not resource-efficient. However, it is often not absolutely necessary to discard the pipette directly after the first use if some treatment fluid for additional uses remains after the first use, and on the other hand the danger of an altered effect or a contamination within the relative short time until the subsequent use is negligible, or respectively apparently nonexistent, at least when it has been properly stored until then.

According to many professional opinions, a pipette with tear replacement liquid can still be used for about 24 hours after being opened by twisting off the sealing cap before the bioburden would become problematic given the predominantly aqueous solutions. Accordingly, tear replacement fluid can be instilled from one and the same pipette into the eye several times a day.

Physicians and pharmacists occasionally recommend wrapping the pipette in aluminum foil after first use for protection. This is, however, impractical to manipulate, and contact between the open pipette tip, or respectively the drop fluid, with the aluminum is to be expected. Moreover, liquid frequently flows out of the pipettes when pressure, for example in the pockets of pants, is exerted on the pipette.

Storage containers for pipettes containing eye drops have also already been proposed. German utility model DE 29508042 U1 discloses a storage container with a cover for plugging or screwing onto the housing opening. The storage container should accommodate a pipette standing upright, wherein the dispensing opening of the pipette is directed towards the cover. Pipette and cover are not interconnected and in particular the pipette is not removed together with the cover.

The subject matter of US 2008/0283530 A1 is of a similar nature. It discloses a two-part storage container that is open toward the top in which the pipette is placed with the dispensing opening upward. The pipette is loosely placed into the lower body of the container and is inserted into the lower body including the dispensing chamber. The pipette is not mounted to the lower body and will fall out if the lower body is turned upside down.

US 1700781 teaches a container in the form of a glass bottle with a rubber lid closing the bottle neck, wherein a glass pipette with two openings is mounted with its upper tubular opening end to the lid by inserting the tubular end into an elastic circular channel formed in the rubber material of the lid. The lid is called a stopper. The circular channel is admitting air to the upper opening of the pipette, the channel ending on the side of the stopper so that the channel is closed by inserting the stopper into the bottle neck. As such the lid is a functional element of the pipette operating as a drip-stopper.

Similar US 2180248 teaches a casing having an upper open end with a cap for closing the casing. The cap permanently carries an elongated rubber bulb that secures the opened upper end of a glass tube which has a restricted lower end to provide an opening in the form of a nozzle. In the closed state the tip of the nozzle rests on a cushioning member arranged in the dead end of the casing. The device is a pocket dispenser for liquid antiseptics where the glass tube acts as a reservoir for the liquid. The liquid is dispensed by pressing the bulb.

### Object of the Present Invention

The object of the present invention is therefore to be able to store a pipette containing a fluid that is safely protected from biological, chemical and physical interference, both in an unopened as well as in an opened or reclosed state. Moreover, the storage option should enable easy handling in general and especially of the pipette, in particular when using a treatment fluid like eye drops, and should extend the length of usability of the fluid in the respective pipette beyond single usage.

### Summary of the Invention

The present invention relates to a storage container for the sealed accommodation of at least one pipette, wherein the pipette is made of a plastic material having one main longitudinal axis (= axially) along which the pipette axially arranged comprises:
- a dispensing opening at one end of the pipette; and
- a tab at the opposite end of the pipette;
- with a dispensing chamber for storage of a fluid therebetween, wherein the dispensing chamber is squeezable for dispensing the fluid through the dispensing opening;
   - wherein the tab has a plate-shaped body, the plate-shaped body comprising two axially parallel edges, and
   - wherein the tab comprises a tab end, the tab end is part of the plate-shaped body and preferably is arranged at the end of the tab remote from the dispensing chamber;

wherein the storage container comprises:
   - a tubular housing having a first opening on its first end, and
   - at least a first cover for removably closing the first opening of the tubular housing, the first cover comprising a clamping fixture formed
      to fix the tab end on the first cover with at least the dispensing chamber completely protruding from the clamping fixture and
      to align the pipette for inserting the pipette into the tubular housing with the one end comprising the dispensing opening first,
      the cover closing the first opening of the storage container while the pipette is attached to the cover,
wherein the clamping fixture comprises one of
   a) one or more spring elements;
   b) a slot comprising one or more non-movable clamping elements that provide a constriction of the slot, where the constriction provides a wedging or a pressing or a deformation, in particular a bending, of the tab end when inserted into the slot;
   c) a toggle lever or screw clamp;
   d) a lance, preferably a flat lance, with a lance tip for insertion of the tip into the tab end;
for retaining the tab end on the first cover.

The clamping fixture attaches the pipette to the cover so that the clamping fixture can hold the weight of the pipette including any fluid therein when the first cover with the pipette attached is lifted while the dispensing opening is downwardly directed.

The clamping fixture according to the embodiments a) and c) may additionally comprise a slot, where the one or more spring elements, the toggle lever or the screw clamp are each arranged to provide a constriction of the slot. The slot preferably has an essentially slit-shaped opening, where the length of the slit preferably corresponds to the width of the tab end (width B).

The cover may have an inner surface surrounded by an annular skirt forming a cavity, the cavity comprising the clamping fixture where the dispensing chamber is completely protruding from the cavity when the tab end is inserted in the clamping fixture, thereby allowing free access to the dispensing chamber in order that a user may squeeze the dispensing chamber with his/her fingers to dispense fluid out of the one opening of the pipette, while the cover is still mounted to the pipette via the clamping fixture.

In particular, the tubular housing of the storage container has the shape of an elongated straight tube.

Preferred embodiments are the subject matter of the dependent claims or are described below.

According to one embodiment, the second end of the tubular housing opposing the first end is permanently sealed by a housing base as an integral component of the housing.

In another embodiment, the tubular housing has a second opening in its second end, wherein a second cover is provided for closing and opening the second opening or, on the other hand, a housing base is provided that is attached to and permanently seals the second opening. The second cover may be designed exactly in the same way as the first cover.

According to one embodiment, on its side facing the second opening in the housing, the second cover has a clamping fixture for the tab end of a pipette for holding the pipette, wherein the pipette is the second pipette in the housing or the first pipette. The clamping fixture may be the same as in the first cover or may be an alternative type of clamping fixture as disclosed herein, e.g. for a differently formed tab end.

If a pipette is attached to the cover and the cover closes the end of the housing, the longitudinal axis of the pipette extends substantially parallel to the longitudinal axis of the tubular housing, wherein the front end of the pipette ends in the open space of the container and as such does not touch any object such as the other end of the housing, a housing base, another cover with or without clamping fixture or the front end of a second pipette.

According to a preferred embodiment, the pipette extends forward when held in a first or second cover, apart from any lateral guides in the interior of the housing, i.e., substantially free-floating at least from the front end of the clamping fixture.

In another embodiment, two pipettes are held substantially parallel to each other by their respective tab end in one or two clamping fixtures of the first cover, wherein the second end of the housing has a housing base. The two pipettes can be inserted into the clamping fixture where the two pipettes are connected to each other in the form of a strip consisting of two pipettes, or can be inserted individually into the clamping fixture.

### Detailed Description of the Invention

The pipette that can be inserted into the storage container according to the present invention is further defined in claim 1 and/or claim 12.

The rear end of the pipette is formed by the tab, and the opposing front end of the pipette is formed by the sealing cap. The dispensing chamber lying in between preferably terminates at its front end with a neck piece that has the dispensing opening, which is closed by the sealing cap and forms the pipette tip to the outside toward the front end. The dispensing chamber is closed at its rear end. Next to the dispensing chamber is the tab. The tab end is defined as the rear region of the tab that is needed by the clamping fixture to hold the pipette, wherein the tab end can also extend up to the front end of the tab. The tab has a plate-shaped body. The body may for example be designed as a flat elongated rectangular cuboid.

The pipettes are made of a plastic material, in particular of polyethylene or of polypropylene.

Generally, the pipettes are designed long and flat. They generally have two parallel longitudinal side edges at least in the region of the tab, and a generally bulging dispensing chamber that elevates the planes on both sides formed by the large area side walls of the tab. The dispensing chamber may hold a content of about 0.1 to 1.5 ml, frequently 0.2 to 0.6 ml, in particular in case of an artificial tear replacement fluid.

The tab can accommodate one or more product identifications. The product identification is generally adhered to the tab in the form of a label. However, an identification can also be stamped into or printed on the tab. At the same time, the tab serves as a grasping aid. The tab does not contain any fluid.

Common to many conventional pipettes on the market is that they possess such a flat, rectangular cuboid end piece for easier handling so that the pipette can be easily gripped. Across many manufacturers, the tab, in particular the tab end, has relatively uniform dimensions with respect to the width (typically 1.2-1.3 cm), thickness (typically 0.2 to 0.4 cm) and length (typically 2 to 3 cm) of the tab with only slight, manufacturer-related deviations. Consequently, a standardized clamping fixture for the tab end is feasible for a large number of different pipettes.

According to one embodiment the plate-shaped tab is hollow. The hollow space may be created by welding the borders of two thin-walled, flat, rectangular half shells together thus forming two essentially parallel walls, hereafter also called large area side walls.

Pipettes with fluid are generally marketed in the form of a plurality of pipettes joined to each other to form a so-called strip. The strip has the form of a sequence of several pipettes connected to each other side by side. For this, the pipettes have edge regions (for example also in the form of connecting bars) that are connected to each other along break lines. The break lines run in particular on both sides along the narrow longitudinal side of the tabs, in particular parallel.

A typical use of a pipette may be explained as follows. If desired, the pipette can be manually shaken downward several times until the liquid is located in the neck piece of the pipette close to the dispensing opening. The sealing cap of the pipette is then twisted off, and the pipette is held between the index finger and thumb over the eye for instillation. By exerting pressure on the dispensing chamber, the treatment fluid exits through the dispensing opening into the eye.

Common to opened pipettes is that they generally do not drain without pressure being exerted on the dispensing chamber, even if they are already partially empty and the dispensing opening hangs downward suspended freely.

With many pipettes, the sealing cap can be placed again on the dispensing opening after being initially removed, and the dispensing opening can therefore be reclosed. However, pipettes are also commercially obtainable where the sealing caps cannot be reused.

According to one embodiment, the sealing cap may have a grip piece and the actual closure. The closure projects on the side of the grip piece facing in the direction of the dispensing opening and is frequently provided with a hemispherical hollow expansion that is suitable for adjoining the annular pipette tip with a narrower, flexible entry hole in the hemispherical base area and to close the pipette tip by the press fit of the hole wall with the neck piece.

The grip pieces make it possible to more easily break off, or respectively twist off the sealing caps without losing them, and to place the sealing caps on the pipette tips after use again. However, given the small dimensions of the sealing caps, handling is usually difficult for the user, and at least very impractical and susceptible to contaminations.

The storage container described herein serves to store a pipette, or one or two pipettes each with a tab. The pipettes can be stored in the container unopened, opened, or reclosed.

The container comprises a housing in the form of an elongated straight tube. At the first end, the housing possesses a first opening which is closed or opened by a first cover.

At its second end, the housing has a housing base as an integral component of the housing, or possesses a second opening there which is permanently closed with a housing base as a separate component, or which is closed or opened with a second cover.

A cover as part of the container according to the invention is intended to be regularly removed, or respectively mounted for opening, or respectively closing the housing. After being assembled with the housing, a housing base is intended to be no longer removed from the housing.

On its side facing the opening in the housing, the first cover has a clamping fixture for the tab end to clamp a pipette on the cover or, in other embodiments, has a wide clamping fixture for up to two pipettes, or two clamping fixtures for one pipette each.

Depending on the embodiment of the container, the second cover can have a clamping fixture for a pipette, or does not provide a clamping fixture and then serves for example only for sealing, or for opening and closing at the second end of the housing during any cleaning of the housing.

A cover with a clamping fixture can be produced as one part or from joined parts. The entire clamping fixture or clamping fixture components can be releasably connected to the cover (for example by a snap-in or screwed connection) or permanently connected (for example by welding, soldering, gluing, press-fitting and deforming).

When the storage container is in a state of use, up to two pipettes can be inserted into the housing, or respectively removed from the housing, through its first opening, and if applicable through the first and second opening, with the assistance of a cover with a single or double clamping fixture (for one pipette or up to two pipettes), or with the assistance of two covers that each have a clamping fixture (for one pipette each). In addition to holding the pipette, the covers also allow the housing to be closed or opened during frequent daily use. The covers can therefore be removed from the housing with or without a pipette and can be moved without the housing. Cover and (tubular) housing are typically not connected.

The pipette is inserted into the housing while the pipette is held in the clamping fixture of a cover preferably by contacting, or respectively placing the pipette neck piece against the edge of the housing opening and then rotating the pipette around the contact line so that the pipette tip is inserted into the housing under a rotary motion, and the longitudinal axes of the pipette and housing are aligned substantially parallel. Afterward, the pipette can be entirely inserted into the housing. Generally, the pipette can also be inserted directly, i.e., without contacting, or respectively placing, by a normally steady hand, wherein the longitudinal axes of the pipette and the housing then substantially correspond from the onset.

The tubular housing has a preferably circular, constant cross-section but can also have any desired shape and variation of the cross-section along the longitudinal direction such as a polygonal cross-section, for example in the form of an octagon, or a cross-section with two longer parallels that are connected to each other by semicircles so that two pipettes can be shoved into the housing next to each other.

Typically, independent from the different designs described above, the storage container has a length within a range of 3 to 15 cm and a width within a range of 1 to 4 cm. In terms of length and width, the storage container is preferably characterized for the different designs by one or more of the following features:
a) The length of the storage container is 3 to 15 cm, in particular 5 to 9 cm, when the storage container is provided for one or two pipettes held in the same cover, or 7 to 15 cm, in particular 8 to 11 cm, when the storage container is provided for two pipettes arranged substantially along the longitudinal axis of the tubular housing.
b) When the storage container only has one clamping fixture for a pipette in the first cover, the width of the storage container is 1 to 3 cm, in particular 1.3 to 2 cm or 1.3 to 2.2 cm when the storage container is single-walled, or respectively 2 to 2.6 cm when the storage container is double-walled.
c) When the storage container has one clamping fixture for two pipettes or two clamping fixtures for one pipette each in the first cover, the width of the storage container is 2 to 4 cm, in particular 2.5 to 3.3 cm when the storage container is single-walled, or respectively 3.3 to 3.9 cm when the storage container is double-walled.

With the embodiment in which up to two pipettes are held in the first cover, the width of the container increases in at least one lateral direction by, for example, about 1.3 cm in comparison to the design with only one pipette in the first cover. The container length instead lies in the lower of the above-stated ranges when the user wishes to only store pipettes without a sealing cap in the container, and/or when he shortens the tab at its rear end to a minimally required remaining length for holding, in particular 0.5 to 1 cm.

According to one embodiment, the container accommodates a single pipette through the opening in one housing end, whereas the second housing end is closed by the housing base as for example an integral component of the housing, or a second cover (if applicable without a clamping fixture).

Instead of a storage container for only one single pipette, a storage container for up to two pipettes is occasionally more practical when for example the content of a pipette is insufficient as a daily dose, or two differently flowing treatment media are to be used. According to one embodiment, the container can accommodate two pipettes for this through the two opposing openings in the tubular housing with the assistance of housing covers with an integrated clamping fixture. Each of the two covers then holds one pipette by means of a clamping fixture, or respectively is designed to hold one pipette each, and closes the housing at the respective end in a way that the cover can be reopened. The design of the two covers and in particular the layout of the clamping fixture can be identical or different for each cover.

For example, the second cover can have basically the same shape (such as a pot shape with an inner thread) as the first cover, but may have no or a different clamping fixture for holding a second pipette. Accordingly, for example in addition to a pipette with a tear replacement fluid, a pipette with a saline solution for rinsing eyes can be stored in the storage container. These pipettes may for example have tab ends of different dimensions.

In an alternative embodiment, two pipettes can also be clamped next to each other directly in the first cover by a wider common clamping fixture or two separate clamping fixtures for two possible tab ends each, wherein the housing cross-section is designed correspondingly larger, and the second housing end has a housing base or a second cover without a clamping fixture. The two pipettes are separate at their narrow longitudinal sides, or connected by their original connecting bars. As the above embodiment, this version also permits just one single pipette to be accommodated in the storage container.

If one pipette is held in a cover in all of the embodiments and the cover closes the associated opening of the housing, the longitudinal axis of the pipette extends substantially parallel to the longitudinal axis of the tubular housing, wherein the front end of the pipette ends in the open space of the container and as such does not touch any object such as the other end of the housing, a housing base, another cover with or without clamping fixture or the front end of a second pipette.

According to a preferred embodiment, the pipette extends forward when held in the cover, apart from any lateral guides in the interior of the housing, i.e. substantially free-floating at least from the front end of the clamping fixture.

By its front side, i.e., on its side facing the opening of the housing, the cover can be shoved, plugged onto, pressed, snapped on or screwed on to its associated opening. In particular, the cover has an inner or an outer thread that always corresponds to an associated thread on the housing end.

The outwardly facing side of the cover with the clamping fixture can be constructed so that the cover can be placed on its associated housing opening, or on the opposing housing end on the housing base or on the second cover, for example in the form of a plug-and-socket similar to that of the cap of a fountain pen.

According to one embodiment, the cover is designed pot-shaped and the clamping fixture for clamping the tab end of the pipette is arranged in the cavity formed by the covers pot interior.

The cover can for example be safely placed with the pipette, with the tab end in the clamping fixture, on a storage surface such as on a table on its side to the outside of the container without the pipette tip touching the storage surface; in particular, for example, a pot-shaped cover lies on its outer cover base surface, wherein the longitudinal axis of the pipette is oriented perpendicular to the storage surface, and the pipette tip is oriented upward.

The risk of the pipette tip contacting the storage surface is therefore less than when handling the pipette without a cover. The stability can be improved by a low center of gravity of the cover close to the cover base. This can be achieved for example with a reinforced cover base of the covers own material, or with an additional element fastened to the cover base, such as in the form of a metal annular disk lying in the cover when the cover is pot-shaped. Moreover, a permanent magnet, in particular a magnet comprising a rare earth metal, can be attached to the cover base that shifts the center of gravity further to the cover base and allows the cover to be attached to everyday objects being ferromagnetic.

During instillation, it is possible to leave the cover on the employed, seated pipette.

The clamping fixture makes use of spring elements and/or the elastic and/or plastic properties of the tab itself. In the latter case, non-movable clamping elements for example deform, wedge or press the tab end when inserted into the clamping fixture thereby fixing the tab end in between the non-movable clamping elements.

If the clamping fixture makes use of spring elements at least one spring jaw may align the tab in the direction of its width B and/or at least one spring jaw may align the tab in the direction of the thickness S so that the longitudinal axis (main axis) of the pipette is aligned in the longitudinal direction of the cover, or respectively in the longitudinal direction of the housing.

Given the minor manufacturer-related deviations of the tab width B, according to one embodiment spring jaw(s) in the direction of the width B can be omitted, and the tab end with its two axially parallel side edges may be guided along two opposing guide surfaces.

The clamping fixture having one or more spring elements may have one spring jaw engaging on one side of the tab with an opposing rigid guide surface or, a pairing of two spring jaws similar to a binder clip engaging on two opposing sides of the tab. A pairing of two spring jaws may be made of a strip of spring material bent into the shape of an isosceles triangle preferably with loops at the apex.

According to one embodiment the spring jaw presses against the tab end without deforming the tab end. According to two another embodiments, the clamping force of the spring jaw deforms the tab end either in an elastic or plastic way, in the latter case for example by a needle-shaped tip on the contact line of the spring jaw with the tab.

The material of the clamping fixture should ideally consist of the same or similar material as the cover material. Using other materials for the spring jaw as the cover material may be considered; these are in particular very strong spring materials such as titanium alloys or high-grade steels.

In order to circumvent the construction of a spring element to be mounted separately in the cover for clamping the tab end, the spring construction can for example be manufactured as an integral component of the cover in an injection molding or extrusion process.

Moreover, to avoid the use of spring jaws in the construction of the clamping fixture or to further support the action of one or more of spring jaws, the properties of the tab being elastic and/or plastic deformable can be used. For example, the tab end can be squeezed, wedged, twisted and/or bent where the respective clamping fixture provides one or more non-movable clamping elements such as constrictions for clamping the tab end. In particular the constrictions can be designed in the directions of the thickness S or the width B or about an axis in the direction of the length L or width B of the tab.

Such clamping fixtures may have the following designs:
According to a first design, the clamping fixture comprises a slot extruded outwardly from the cover base and having a rectangular cross-section. The slot has an opening that is adapted to the cross-section of the tab end. At least one large area wall of the slot provides a clamping constriction in the direction of the thickness S of the tab, for example at least one lateral protrusion that is positioned on one of the large area walls of the slot for example approximately in the middle. The constriction is smaller than the thickness S of the tab causing a deformation or pressing of the large area side walls of the tab in touch with the lateral protrusion, resulting in a clamping constriction that fixes the tab in the slot and thereby holds the pipette.

According to a further embodiment of the first design, one of the large area walls of the slot has a lateral protrusion extending into the cavity of the slot being located approximately in the middle of the width of one of the large area walls of the slot, while opposite to said first large area wall of the slot there is provided a second large area wall having a guide surface and an opening in the second large area wall. The slot has again an essentially rectangular cross-section. When the tab end is inserted into the slot the tab end bents, due to the action of the protrusion in the direction of the thickness S, into the open space provided by the opening. The bending causes a friction force that firmly holds the tab end in the clamping fixture.

According to a second design, a retaining force can be generated by elastically bending the tab about an axis parallel to the direction of the tab width B. The cover has a slot in the form of an extrusion, the extrusion providing a pathway having an essentially rectangular cross-section and an arc-shaped curvature, so that the walls of the extrusion exert a bending force on the tab end when inserting the tab into the slot. This provides a friction lock against the large area side walls of the tab that firmly holds the tab end in the clamping fixture.

According to a third design, a slot is provided with two opposing walls that are tapering towards the rear end of the slot applying a wedge-shaped constriction on the tab end, while optionally the remaining surfaces of the slot provide opposing guide surfaces.

According to one embodiment of this third first design, the slot is provided in the form of an extrusion having an essentially rectangular cross-section where the small area walls of the slot provide a tapering, in particular at the rear end, forcing the tab end to be bent about its longitudinal axis or to be squeezed in the direction of its width B. The tab acts similar to a leaf spring pushing the side edges of the tab end against the tapering walls, thereby applying a retention force.

According to a fourth design, the slot, similar to the second design, can force the tab end to bend around several protrusions or curvatures, optionally in opposing directions (around axes parallel to the direction of the width B). If for example a straight hollow cuboid forming the slot is extruded out of the cover base and if the two large parallel sides are provided with protrusions alternating on opposing surfaces in the interior of the slot (i.e., in the direction of the tab thickness S) then a wavy or S-shaped pathway for the tab end results and the tab end is clamped by a friction lock due to the bending forces generated by the protrusions or the S-shaped pathway.

According to a fifth design the slot has the shape of a hollow cylinder with two azimuthally arranged wedges on the inner cylinder wall for a radial tapering (for example positioned at approximately 10°-170° and 190°-350°), dedicated for the side edges of the tab end. The tab is smoothly inserted into the slot in longitudinal direction at approximately 0° between the two wedges. Afterwards the tab is turned about its longitudinal axis until the side edges of the tab clamp tightly to the wedge walls. If the tab is turned any further then the clamping force is increased by bending or compression of the tab, as described above. To avoid an unintentional release of the clamped tab by vibrations or handling of the pipette, the wedges can have a roughened or wavy surface, a radially not changing end or a notch or groove at one or more locking positions for the tab.

A sixth design addresses pipettes with a cut-off end at the rear end of the tab that allow to push the tip of a flat lance into the hollow space of the tab at the tab end. The lance may have the form of a thin plate with a tapered tip. The lance stands up vertically on the inner side of the cover base. The lance is inserted into the hollow interior of the tab end and expands the two large area side walls of the tab. This expansion exerts a friction force on the large area side walls of the tab, thereby clamping the tab end onto the cover.

According to a further design the clamping fixture comprises a hollow rectangular cuboidal slot for inserting the tab end and one of a toggle lever or a screw clamp. The toggle lever or the screw clamp is mounted in / onto one of the large area walls of the slot and provides an insertable actuating element reaching into the cavity of the slot for pushing the tab end laterally against the opposing large area wall. The screw clamp may additionally comprise a pointed screw which on turning the screw moves forward and presses into the tab end and at the same time pushes the tab against the opposing large area wall.

As described before, clamping fixtures may extend into the interior of the container or may face outwardly relative to the cover base.

A clamping fixture can also be manufactured as a part or as a combination of parts separate from the main body of the cover that is fastened to the cover later on.

Preferably the storage container should be light and small so that handling such as wearing or using the pipette remains comfortable for the user. In this regard, reinforcements can minimize the risk of bulging in the case of thin container walls. If, for example when the housing has a circular cross-section, these reinforcements extend into the interior of the housing in the form of radially pressed-in circular grooves then they can simultaneously serve to better guide and remove, or respectively insert, the pipette when the inner diameter is adapted. Moreover, such grooves can serve to fix the overall construction of the container on parts of clothes or bags with a wearing device, for example in the form of an additional clip.

A double or multiple-walled storage container can provide thermal insulation whose insulating strength can be influenced in particular by the selection of a container material with low thermal conductivity (such as high-grade steel or titanium alloy instead of aluminum) and effective insulating material (such as foam, powder, fibers, powder with added metal, or multilayer insulation) and by a vacuum.

In one embodiment, the storage container is constructed double-walled with an enclosed vacuum. When insulating such a double-walled container with a vacuum (in the space between the outer and inner wall), the pressure lies within the range of the high vacuum and frequently requires the use of getter or dryer materials for gas molecules, or respectively steam, for long-term stabilization.

In another embodiment a material is filled between the walls with an insulating material for example an open pore material. The filled space is either not evacuated or has a higher pressure level of the vacuum than the previous embodiment.

Additionally, it is possible to use coolants (for example with so-called phase change materials) close to the inner wall of the container (for example inserted in the container interior or in an additional sleeve-shaped wall).

The housing and the cover, or respectively covers, preferably have a hard surface in order for example to avoid scratches from other objects in the handbag, or also when cleaning with brushes. For example, anodic oxidation of the surface (anodizing) is useful when using aluminum or its alloys.

Other metals with a high tensile strength in comparison to aluminum or aluminum alloys can further increase the hardness of the surface. In this case for example, titanium and its alloys and high-grade steels can be considered possible container materials. Depending on the material, other hardness enhancements besides anodization can be achieved, for example by heat treatment, introducing foreign atoms, applying protective layers or by work hardening. The anodic oxidation of titanium or titanium alloys simultaneously makes it possible to color the container made of this material.

For challenging environments, preferably the storage container should be watertight and corrosion-resistant so that it can be used, for example, in the rain, while swimming in an indoor pool, in the ocean, or for cleaning with boiling water or in the dishwasher.

The storage container is preferably hermetically sealed from the environment when the cover(s) is/are mounted. For impermeability, a sealing ring made of plastic can, for example, be mounted either on the cover or on the housing.

The housing and/or the cover can consist of metal or plastic, in particular titanium or a titanium alloy. Titanium as well as titanium or high-grade steel alloys have proven to be very suitable as regards corrosion resistance. Plastics such as polyethylene, polypropylene or polyamides are a more economical alternative. Certain aluminum alloys can offer adequate corrosion resistance for many applications.

Several storage containers can be linked into a bundle, for example by inserting them into several elastic loops.

The present invention will be explained in greater detail with reference to the following figures without being restricted thereto:

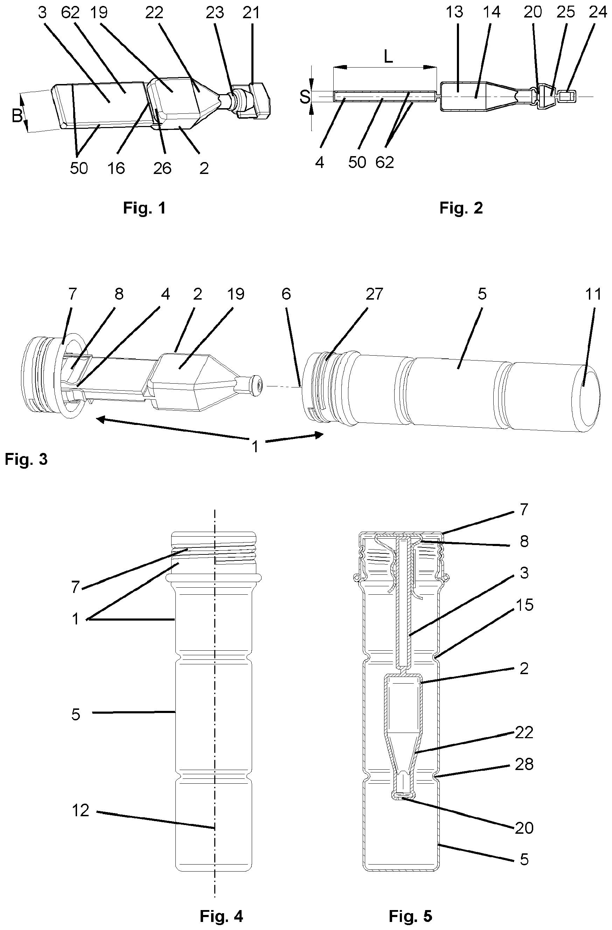

Fig. 1 shows a pipette 2 in a three-dimensional view, and Fig. 2 shows the same pipette in a sectional view in a longitudinal direction 14. Arranged sequentially, the pipette has: a flat graspable tab 3, a dispensing chamber 19 in which a fluid 13 is located, a dispensing opening 20 and a sealing cap 21 on the dispensing opening. The pipette is designed in a bulging manner approximately in the middle where the dispensing chamber 19 is and constricts toward the dispensing opening 20. The constricted part of the dispensing chamber is called the neck piece 22. The end of the neck piece is termed the pipette tip 23. The sealing cap 21 has a grip piece 24 for disconnecting the sealing cap from the pipette, and a hollow hemisphere 25 for resealing the pipette.

At its rear end 26, the dispensing chamber 19 transitions into the front end of the tab 16. Relative to the dispensing opening 20, the tab forms the opposite end of the pipette having a tab end (4) at the end of the tab.

The portrayed tab has the shape of a thin elongated cuboid with a width B, a length L and a thickness S. The tab is hollow and accordingly has two large area side walls 62 that can be elastically pressed in the direction of the thickness S. The tab is moreover elastically deformable about an axis parallel to the direction of the width B being the distance between the two side edges 50 and about an axis in direction of the length L.

Fig. 3 shows a storage container 1 comprising a tubular housing 5 with a round cross-section, the first end of which has an outer circumferential screw thread 27 and a first opening 6, and the second end of which is sealed by a housing base 11. A removable cover 7 can be placed on the screw thread while rotating, wherein the cover contains a clamping fixture 8 on the inside of the cover base 33 in which the tab end 4 of a pipette 2 is inserted, wherein it is tightly clamped. The cover 7 has a pot-shape with an inner thread and is screwed off from the housing 5. The pipette 2 is opened and is removed from the housing 5.

The pipette is shown in a side view (narrow longitudinal side of the tab), wherein the dispensing chamber 19 is easily visible.

Fig. 4 and its sectional view in Fig. 5 show the storage container 1 from Fig. 3 with a cover 7 placed thereon, wherein the cover has been screwed onto the outer thread of the tubular housing 5. The pipette 2 is held securely by the clamping fixture 8 in the storage container 1, wherein the pipette is additionally aligned parallel to the longitudinal axis 12 of the housing by a first circumferential, radial constriction 15 of the housing in the region of the tab 3. The constriction accordingly serves as a lateral guide. The second circumferential constriction 28 then only has the function of mechanically stabilizing the housing wall. The bottom end of the neck piece 22 and the dispensing opening 20 are spaced away on all sides from the inner wall of the tubular container 1. The dispensing opening 20 is not sealed. Nonetheless, no fluid drains out.

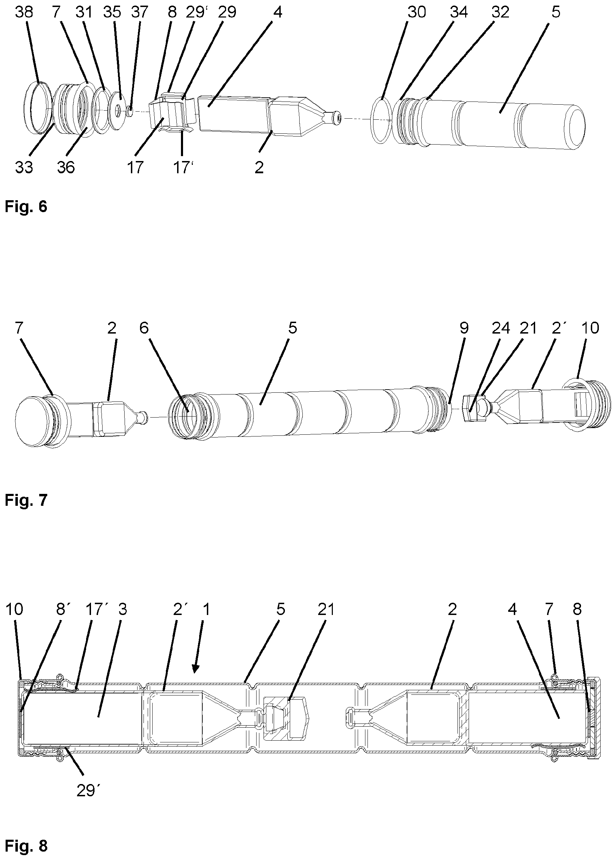

In an exploded view, Fig. 6 shows a storage container 1 with a tubular housing 5, cover 7, clamping fixture 8 and pipette 2. The clamping fixture 8 has in each case a pair of spring jaws 17 and 17' and an associated guide stop 29 and 29'.

As an expansion of the above embodiments from Fig. 3 to Fig. 5, two sealing rings are shown in the embodiment according to Fig. 6: one sealing ring 30 can be shoved onto the housing up to the stop bead 32 of the housing 5 for the cover opening edge. Another alternative sealing ring is inserted as a flat sealing ring 31 in the cover base 33 against which the edge of the housing opening 34 is pressed when screwing in the cover 7.

As another expansion of the above embodiments from Fig. 3 to Fig. 5, an annular metal disk 35 according to the embodiment in Fig. 6 is fastened at the inside of the cover 36 to the cover base 33 to which in turn the clamping fixture 8 is fastened. The metal disk 35 serves as a weight for very light cover constructions so that the cover 7 with the inserted tab end 4 and the pipette 2 held therewith can be stably placed on a storage surface by its cover base 33. Moreover, the metal disk accommodates a small permanent magnet 37 in the center and amplifies its magnetic field toward the outer cover base side. Accordingly, the cover or the entire container can adhere to ferromagnetic components, for example in a bathroom or office.

In order to shield the magnetic field to the outside when carrying the container 1 in a sensitive environment, an additional ferromagnetic cap 38 is placed on the outside of the cover base 33 in this embodiment.

Fig. 7 and 8 show a storage container 1 with a housing 5 in the form of a tube open at both ends with two covers 7, 10 that can be screwed on for the first 6 and second 9 opening. By its tab end 4, a pipette 2, 2' is inserted into each of the clamping fixtures 8, 8' of both covers; one pipette 2 thereof is in an open state, while the sealing cap 21 of the other pipette 2' has not yet been twisted off. The grip piece 24 is also visible as part of the sealing cap 21.

Fig. 8 shows a section of the container 1 according to Fig. 7. The tab end 4 is in each case clamped in a clamping fixture 8, 8' in the cover 7, 10 that has two spring jaws 17 and 17' as in Fig. 6. A spring jaw presses the tab end 4 against a flat guide surface of the clamping fixture against which the tab rests and that aligns the tab and hence the pipette in the direction of the rotational axis of the screw cover. The flat surface of the clamping fixture functions as a guide stop 29' for the expanded spring jaw 17'.

To improve carrying comfort, the housing length can be reduced by shortening the length L of the tabs at the rear end before using the container (for example with a pair of scissors), in particular to a remaining tab length of 0.5 to 1 cm. A further reduction of the housing length would be possible if the user wishes to use one or both pipettes without a sealing cap.

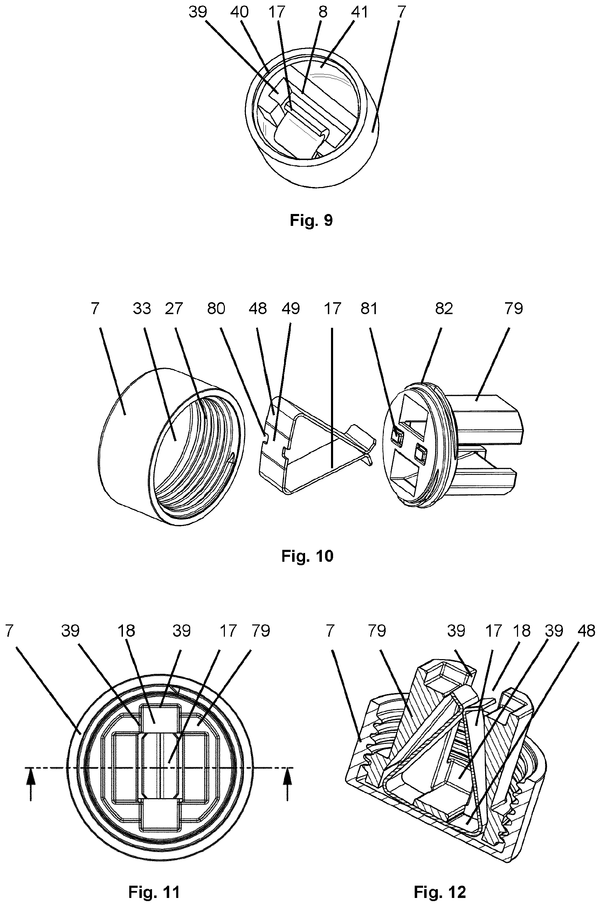

Fig. 9 shows a cover 7 in a three-dimensional view that is designed for a snap-lock with its associated end of the housing. A slotted clamping fixture 8 is discernible that clamps tab end 4, which is held by spring jaw 17. If the tab end 4 is inserted, the tab end 4 slides past the narrowest point of the clamping fixture, and the spring jaw 17 bends slightly backward under spring tension and then lies tightly against the tab. The guidance in the direction of the tab width B is provided without a spring by flat guide surfaces 39. On the inner cover side wall 41, the cover possesses an annular constriction 40 for the snap connection with the housing.

Fig. 10 shows an exploded view of a cover 7 comprising as part of the clamping fixture 8 a spring 48 with two spring jaws 17, a spring support 79 and a slot 18. For the assembly, first the spring is inserted into the spring support 79 from the rear end of the spring support 79 and centered by pairs of centering grooves 80 and rectangular centering protrusions 81 at the bases of the two parts. Afterward this subassembly is screwed with thread 82 into the cover 7, down to the cover base 33 where the spring base 49 is clamped; Fig. 11 and 12 show this final position in a top view and a spatial sectional view through the indicated plane in Fig. 11. The spring support 79 serves, i.a. as a screwing aid for the subassembly, as a guide for the tab end 4 at the guide surfaces 39, as a weight for securely placing the cover base 33 on a storage surface and as restriction to avoid too large displacements of the spring jaws 17. The thread of the cover 27 is also used for closing the housing 5 of the storage container 1.

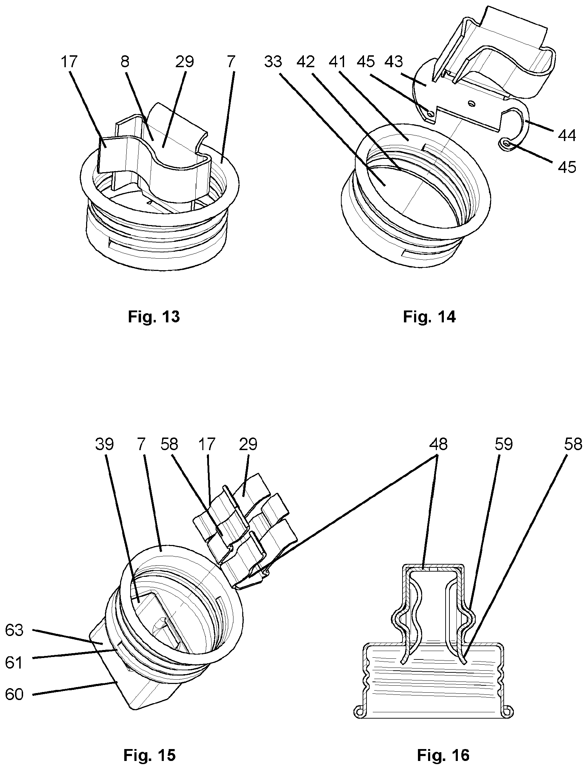

Fig. 13 and 14 show a modification of the clamping fixture 8 according to Fig. 3 to 8. Contrary thereto, clamping the narrow tab side is omitted. In addition, a spring jaw 17 with a bending plane that is parallel instead of vertical to the cover base 33 is used to fix the tab in the clamping fixture 8. When the curved spring jaw 17 is pulled away slightly at its end from the guide stop 29, for example with a thumb, the tab end 4 of the pipette can be inserted almost resistance-free into the clamping fixture 8. Then the spring jaw 17 is released again and snaps onto the large area side walls 62 of the pipette 2 and thereby fixes the pipette in the cover 7. Optionally, an additional needle-shaped tip on the spring jaw that lies in the region of the contact line between the spring jaw and the tab can fix the pipette more securely by drilling the tip into the tab.

To fasten the clamping fixture on the cover base 33, the inner cover side wall 41 is provided with a circular circumferential groove 42. The two lateral wings 43, 44 of the base of the clamping fixture engage in this groove. One wing 43 is shaped like a circle segment, and the other wing is shaped according to the principle of an arm of a circlip (also termed a Seeger ring) 44. Alternatively, but not shown in the present case, the circle segment can be replaced by a second arm in a point-symmetrical arrangement (of the first arm to the cover base midpoint). The clamping fixture 8 is inserted in the cover 7 with conventional forceps whose fingers engage in the two auxiliary holes 45 of the clamping fixture.

Fig. 15 and 16 show a pot-shaped cover with slot provided by a cuboidal extrusion 63 forming a slot. The larger width in the rectangular opening of the slot is adapted to the width of the tab end 4; two associated guide surfaces 39 being the large area wall of the slot correspondingly guide the tab end 4 in this direction. The slot accommodates a spring 48 as a tab holder whose two middle spring elements 58 lock into the two small lateral bulges in the large area walls 59 of the slot by means of a snap connection. By pressing together the ends of these spring elements 58, the entire spring can be easily removed again from the cover 7, for example for cleaning. The two lateral pairs of the spring element consisting of the spring jaw 17 and guide stop 29 are designed with a shape analogous to the above designs and align the pipette 2 in the direction of the rotational axis of the cover. The straight guide stop 29 can be replaced by a curved spring jaw 17. An advantage of the slot type construction in comparison to the constructions according to Fig. 10 to 14 lies in the high rigidity of the clamping fixture under torsion from the pipette, for example when twisting off the sealing cap 21, since the torque is primarily absorbed by the cover wall and not by the spring element 48. Additional advantages result from simple sealing with a flat sealing ring 31 in the cover base, easier screwing on in the case of a screw cap, easier pulling off the cover when there is a snap-lock, and from potentially placing the cover with a mounted pipette on the table where the cover lies on one of the wide edges of the topmost surface 60 of the extrusion and a point 61 on the edge of the outer circular cover base.

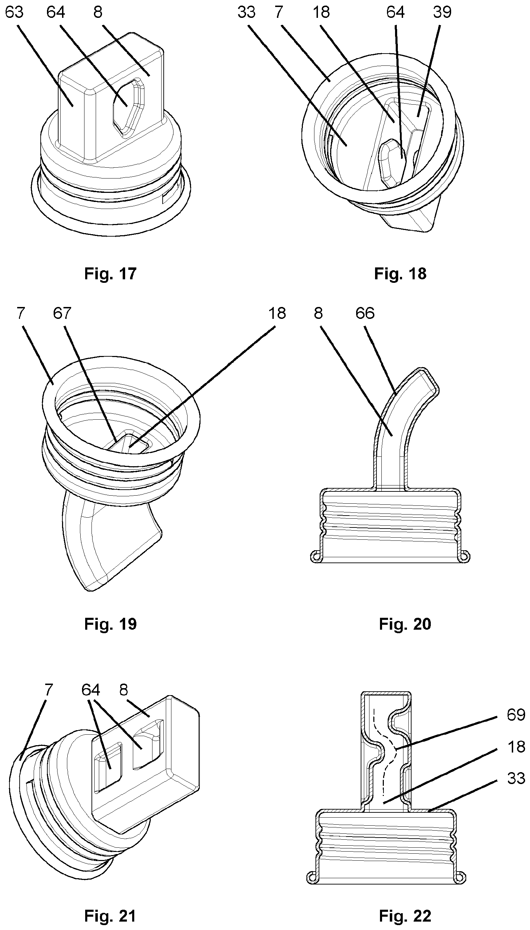

Fig. 17 and Fig. 18 show a slot 18 as the clamping fixture 8 in the cover 7 making use of the spring effect of a bended large area side wall 62 of the tab without the need for using other spring elements as in the above embodiments of the cover. As with the above approach from Fig. 15 and Fig. 16, the cover base 33 has a cuboidal extrusion 63 that is closely adapted to the maximum sectional dimensions of the tab cross-section. Protrusions 64 such as beads are incorporated in one or both of the large area walls of the extrusion and point into the interior of the cover providing a constriction for the tab. The spacing of the protrusions 64 is smaller than the tab thickness S so that the protrusions 64 press into the large area side walls 62 of the tab end 4 when the tab end 4 is shoved into the slot 18. The wedge shape of the protrusions reduces the resistance of the rear tab edge when inserting the tab, and also reduces the danger of small bits of a possible label on the tab being torn off. The latter can be avoided by using a single protrusion instead of two protrusions, or respectively beads.

Fig. 19 and Fig. 20 show a cover 7, wherein the property of the tab 3 is exploited in that the tab end 4 is flexible about an axis parallel to the direction of the tab width B similar to a leaf spring. Accordingly, the clamping fixture 8 in this case is a slot 18 with an arc-shaped curved extrusion 66 on the pot cover base. The base has a rectangular opening 67 which is adapted to the dimensions of the tab cross-section for a smooth fitting. The tab end 4 is inserted there and forces the tab 3 to curve. The curve generates a bending force which presses the tab against the extrusion wall. From the bending force, friction is exerted on the tab wall which holds the pipette in the clamping fixture 8.

According to another embodiment of the cover construction shown in Fig. 21 and Fig. 22, the clamping fixture 8 in the form of a slot 18 is placed boxlike on the cover base 33. The wavy passage 69 in the box interior formed by protrusions 64 in the box walls has three opposing curves so that the tab end 4 is clamped in the wavy passage 69 while being inserted. In this case, two clamping mechanisms are combined with each other: the spring effect of the large area side walls 62 (as in Fig. 17 and Fig. 18), and the spring effect of the tab from being bent several times about the axes in the direction of the spring width B.

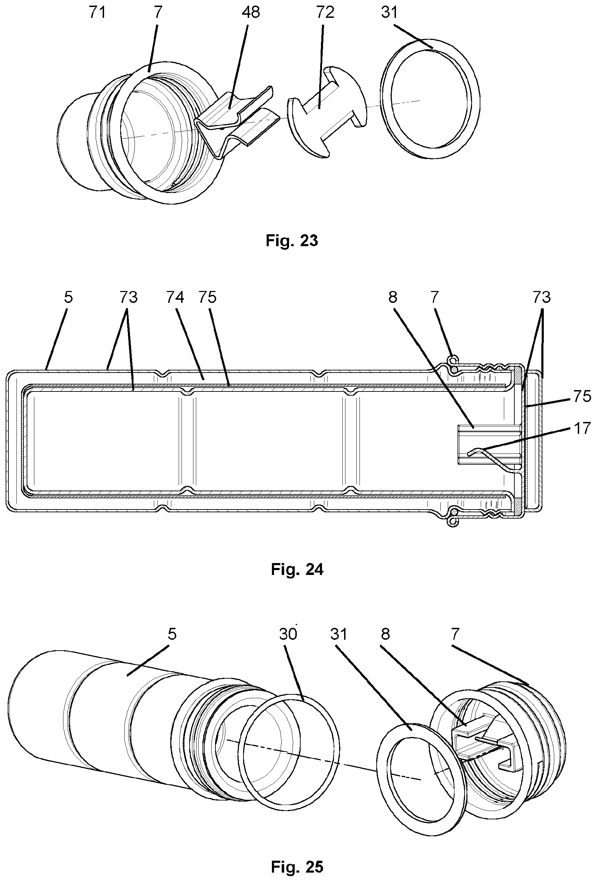

Fig. 23 shows a variation of the clamping fixture 8 with a spring element in which the spring 48 can, for example, be shaped from a simple sheet metal strip. The diameter of an additional cylindrical extrusion 71 in the cover base 33 is adapted to the tab width and therefore centers the pipette 2 in a corresponding transverse direction.

The spring 48 is for example glued, soldered or welded to the cover 7. Alternatively, to this bonding method, an additional element 72 is used in this case in order to press the spring 48 into the additional cylindrical extrusion 71. The flat sealing ring 31 shown lies on the cover base in front of the additional extrusion.

Fig. 24 to 26 show an embodiment of the storage container 1 that enables use in a hotter or colder environment over a longer period by thermally insulating the inner space of the container.

For insulation, a double wall 73 of the container is constructed with an interjacent vacuum 74 as shown in Fig. 24 as a sectional view. Two walls each for the housing 5 and the cover 7 are welded to each other.

To reduce heating from radiation, a super insulation (multilayer insulation) 75 is used between the inner and outer walls of the housing and cover. Thin stacked metal-coated films are suitable for this, also in combination with incorporated microparticles for scattering or absorbing infrared radiation. Alternatively, pure aluminum foils can be used that, for example, are separated by thin fabric as spacers. In addition, the metal surfaces of the container can be polished or reflectively coated by vapor deposition.

The greater diameter of the container required for the insulation is exploited in this embodiment in order to compactly construct the cover as a part, being for example impact extruded, with inner thread as shown in Fig. 25. In so doing, the spring jaw 17 of the clamping fixture 8 is shaped in a separate method step.

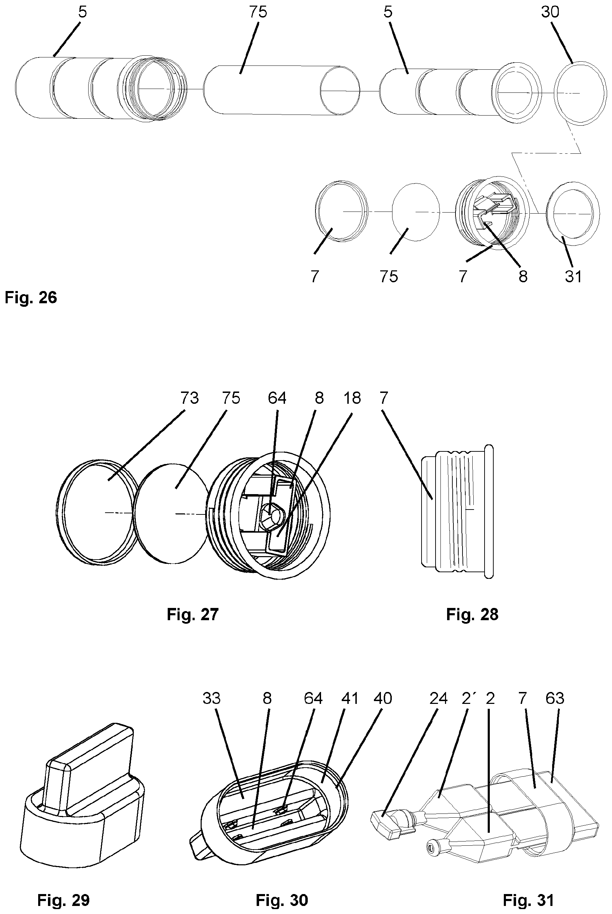

Fig. 26 again shows the individual components of this embodiment of a thermally insulated container in an exploded view.

Fig. 27 and 28 show a cover with only one protrusion 64 formed in the large area wall of slot 18 similar as described for Fig. 17, however, instead of using a curved spring jaw 17 as in Fig.26. Due to an opened opposing large area wall, a bigger protrusion 64 can be designed in order to bend the tab about its longitudinal axis and thereby increasing the frictional retaining force.

Fig. 29 to 31 show a cover 7 that possesses a wide clamping fixture 8 for holding one or two pipettes 2, 2'. Clamping is accomplished with the assistance of protrusions 64 in the cuboidal extrusion 63 from the cover base 33 forming one large slot 18 similar as already described in Fig. 17. On the inner cover side wall 41, the cover possesses a constriction 40 for the snap connection with the housing. If two pipettes are seated, they lie in this cover on one of their narrow longitudinal sides directly adjacent to each other as shown in Fig. 31. In doing so, they can be separate from each other or connected to each other with connecting bars as strips. Fig. 31 shows the first pipette 2 opened and the second pipette 2' unopened. The unopened pipette does not prevent the dispensing chamber of the opened pipette from being pressed. Instillation of the treatment fluid is carried out unhindered as long as the grip piece 24 is not excessively long.

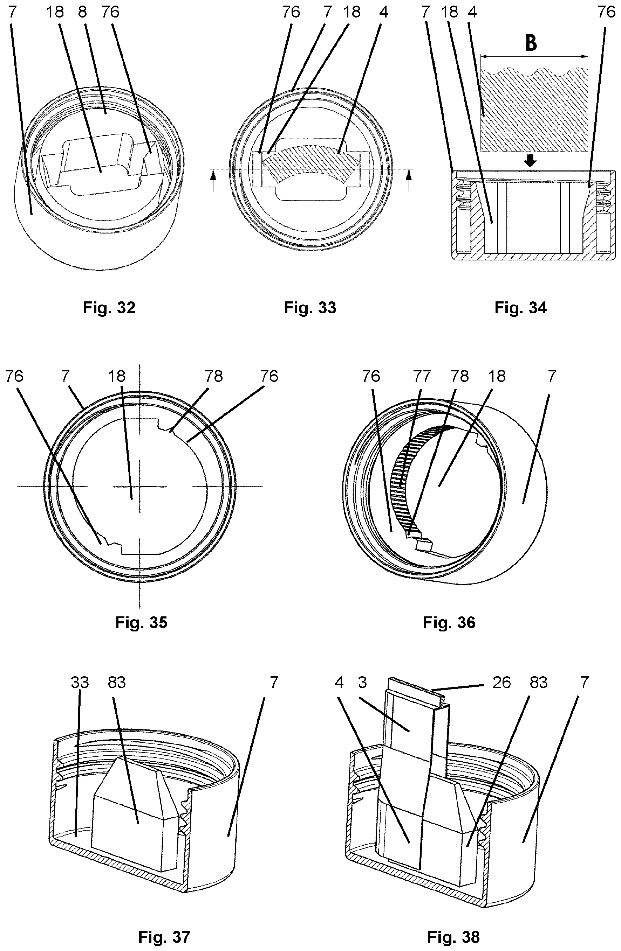

Fig. 32 to 34 show a cover 7 with a slot 18 that has a constriction with a tapering 76 for compressing and/or bending the tab end 4 in the direction of its width B and/or about its longitudinal direction while the tab end 4 is inserted into the clamping fixture 8. Fig.34 depicts a sectional view of the cover 7 and the tab end 4 with a bold arrow that indicates the direction in which the tab end 4 is pushed into the clamping fixture 8, down to the bottom of the cover 7. The constriction has a straight rear end to avoid a slipping back of the tab end 4. The width of the slot 18 is smaller than the width B of the tab end 4 and causes the above referenced deformation with which the side edges 50 of the tab exert forces on the walls of the straight rear end of the slot 18, similar to a leaf spring. This in turn creates a friction force, keeping the tab 3 tightly in the clamping fixture 8. Fig. 33 shows the top view of the cover 7 and its clamping fixture 8, including a dashed region in the center of the view that shall demonstrate the section of a bended tab end 4.

Fig. 35 and Fig.36 show a cover 7 with a slot that has two azimuthally arranged wedges with a radial tapering 76. The tab end 4 is inserted smoothly into the widest part of the slot (i.e. in the vertical position with respect to Fig.35) and is clamped at its side edges 50 by turning the pipette about its longitudinal axis. Beside this wedging effect, a not intended release of the clamped tab end 4 is prevented by a wavy surface 77 of the wedge walls, a further turning of the tab end 4 beyond the first wedging point accompanied by a leaf spring effect due to deformations as described for Fig. 32 to 34, by a small (or no) tapering angle at the end of the wedges, and/or a notch or groove 78 at desired locking positions of the tab end 4 on the wedges.

In the break view, Fig. 37 shows a cover 7 with a flat lance 83 for clamping a tab 3 of which the edge at its rear end has been cut off, thereby allowing access to the hollow space between the large area side walls 62 of the tab 3. The clamping fixture is built as a thin plate with a tapered tip that stands up vertically on the inner side of the covers base 33. The lance 83 fits into the hollow interior of the tab end 4 and expands the two large area side walls 62 of the tab when the lance slides into the tab end 4. This expansion of the tab end 4 exerts a friction force on the large side area walls 62, thereby clamping the tab in/on the clamping fixture 8. Fig.38 shows the inserted tab end 4 in a broken part view, starting at the one end of the tab neighboring the dispensing chamber 26 and cut to one lateral half.

## Claims

1. A storage container (1) for sealed accommodation of at least one pipette (2),
wherein the pipette (2) is made of a plastic material having a main longitudinal axis (14) along which the pipette (2) comprises:
- a dispensing opening (20) at one end of the pipette; and
- a tab (3) at the opposite end of the pipette (2);
- with a dispensing chamber (19) for storage of a fluid (13) therebetween,
wherein the dispensing chamber (19) is squeezable for dispensing the fluid (13) through the dispensing opening (20);
- wherein the tab (3) has a plate-shaped body, the plate-shaped body comprising two axially parallel side edges (50);
- wherein the tab (3) comprises a tab end (4), the tab end (4) is part of the plate-shaped body;
wherein the storage container (1) comprises:
- a tubular housing (5) having a first opening (6) on its first end; and
- at least a first cover (7) for removably closing the first opening (6) of the tubular housing (5), the first cover comprising a clamping fixture (8) formed
- to fix the tab end (4) on the first cover (7) with at least the dispensing chamber completely protruding from the clamping fixture (8) and
- to align the pipette for inserting the pipette (2) into the tubular housing (5) with the one end comprising the dispensing opening (20) first,
the cover (7) closing the first opening (6) of the storage container (1) while the pipette (2) is attached to the cover (7),
wherein the clamping fixture (8) comprises one of
a) one or more spring elements (17);
b) a slot (18) comprising one or more non-movable clamping elements providing a constriction of the slot (18), where the constriction provides deformation or wedging of the tab end (4) when inserted into the slot;
c) a toggle lever or screw clamp;
d) a lance (83) with a lance tip for insertion of the tip into the tab end (4).

2. The storage container (1) according to claim 1, wherein the at least first cover (7) and the clamping fixture (8) are configured so that the dispensing chamber (19) of the pipette (2) can be squeezed with the fingers to dispense fluid out of the dispensing opening (20) of the pipette (2) while the tab end (4) of the pipette (2) is inserted into the clamping fixture (8).

3. The storage container (1) according to claim 1 wherein the clamping fixture (8) comprises a slot (18) and any of a spring element (17), a toggle lever or a screw clamp, wherein the slot (18) preferably has a slit shaped opening.

4. The storage container (1) according to at least one of the preceding claims, wherein the cover (7) has a cover base (33), the cover base (33) has an inner surface surrounded by an annular skirt forming a cavity, the cavity comprising the clamping fixture (8), wherein the dispensing chamber (19) is preferably completely protruding from the cavity when the tab end (4) is inserted in the clamping fixture (8).

5. The storage container (1) according to at least one of the preceding claims, wherein the tubular housing (5) has, on its second end:
a) a second opening (9) and the storage container (1) has a second cover (10) for closing and opening the second opening (9), or
b) a housing base (11),
wherein on its side facing the second opening (9) in the housing (5) the second cover (10) preferably has another clamping fixture (8') for the tab end (4) of a further pipette (2, 2') for attaching the further pipette (2, 2') to the second cover.

6. The storage container (1) according to at least one of the preceding claims, wherein the first end and the second end of the housing (5) oppose each other along the longitudinal axis (12) of the housing.

7. The storage container (1) according to at least one of the preceding claims, wherein the first cover (7), or the first cover (7) and the second cover (10), is/are removable from the housing (5) with the pipette (2, 2') attached to the clamping fixture (8, 8') or the pipette removed from the clamping fixture (8, 8') and wherein the first cover (7) and/or the second cover (10) is/are freely movable without the housing (5).

8. The storage container (1) according to at least one of the preceding claims, wherein the length of the storage container is 3 to 15 cm, and the width is 1 to 4 cm.

9. The storage container (1) according to at least one of the preceding claims, wherein the clamping fixture (8) comprises one or more spring jaws (17) for holding the tab end (4) in a clamping manner, and the clamping fixture (8) optionally further comprises at least one guide stop (29) and/or at least one guide surface (39).

10. The storage container (1) according to at least one of the preceding claims, wherein the clamping fixture (8) is designed in the form of a slot (18), wherein the tab end (4) is insertable into the slot (18) in a clamping manner, and the slot (18) preferably
a) has a wavy or S-shaped passage (69) in the insertion direction,
b) is curved once in an arc form,
c) has at least one protrusion (64) in or on the large area wall of the slot providing a constriction,
d) narrows toward the rear end of the slot (18) in a clamping manner or towards a rotational endposition.

11. The storage container (1) according to at least one of the preceding claims, wherein the housing (5) and optionally the cover (7) is double-walled and/or thermally insulating.

12. A set comprising at least the storage container (1) according to at least one of the preceding claims and at least one pipette (2),
wherein the pipette (2) is made of a plastic material having a main longitudinal axis (14) along which the pipette (2) comprises:
- a dispensing opening (20) at one end of the pipette; and
- a tab (3) at the opposite end of the pipette (2);
- with a dispensing chamber (19) for storage of a fluid (13) therebetween,
wherein the dispensing chamber (19) is squeezable for dispensing the fluid (13) through the dispensing opening (20);
- wherein the tab (3) has a plate-shaped body, the plate-shaped body comprising two axially parallel side edges (50);
- wherein the tab (3) comprises a tab end (4), the tab end (4) is part of the plate-shaped body, and
wherein the at least one pipette (2) is attached to the first cover (7) by the tab end (4) being inserted into the clamping fixture (8).

13. The set according to claim 12, wherein the tab (3) including the tab end (4) have the shape of a flat rectangular cuboid.

14. The set according to at least one of claims 12 or 13, wherein the storage container (1):
a) contains one pipette (2) parallel to the longitudinal axis (12) of the housing (5), and the housing (5) is closed by the cover (7) and by a housing base (11) or by two covers (7, 10), wherein at least one cover (7) has a clamping fixture (8) for clamping the tab end (4) of the pipette (2); or
b) contains two sequential pipettes (2, 2') along the longitudinal axis (12) of the housing (5) and two covers (7, 10) close the housing (5), each having a clamping fixture (8, 8') for clamping the tab end (4) of the respective pipette (2, 2'), wherein the clamping fixtures (8, 8') may be different; or
c) contains two pipettes (2, 2') parallel to each other and parallel to the longitudinal axis (12) of the housing (5), and the housing (5) is closed by a cover (7) and a housing base (11) or by two covers (7, 10), wherein the first cover (7) has a clamping fixture for clamping the tab ends (4) of up to two pipettes or two clamping fixtures for the tab end (4) of one pipette each, and wherein the second cover (10) does not comprise a clamping fixture.

15. The set according to at least one of claims 12 to 14, wherein
- the first cover (7) closes the opening (6) of the storage container (1),
- the longitudinal axis (14) of the first pipette (2) is aligned substantially parallel to the longitudinal axis (12) of the tubular housing (5) of the storage container (1),
- the pipette (2) is arranged in a substantially free-floating manner in the storage container (1) with the dispensing opening (2) spaced apart from the inner walls of the storage container (1).

16. The set according to at least one of claims 12 to 15, wherein the dispensing chamber (19) contains 0.1 to 1.5 ml, in particular 0.2 to 0.6 ml, of a fluid for dispensing through the dispensing opening (20) by squeezing the dispensing chamber (19).

17. The set according to at least one of claims 12 to 16, wherein the front end of the pipette (2) opposite to the tab (3) is formed by a sealing cap (21) that closes the dispensing chamber (19), wherein preferably the sealing cap (21) is discarded in the context of first use or is put on again after being removed.

18. The set according to at least one of claims 12 to 17, wherein the pipette (2, 2') is made of a plastic material, in particular two joined flat plastic materials, in which at least the dispensing chamber (19) is shaped in the form of two half shells, and - also independent thereof - the tab (3) is formed by two plane-parallel flat sections or by two flat half shells.

## Patentansprüche

1. Aufbewahrungsbehälter (1) zur verschlossenen Aufbewahrung zumindest einer Pipette (2), wobei die Pipette (2) aus einem Kunststoffmaterial gefertigt ist, das eine Hauptlängsachse (14) aufweist, entlang der die Pipette (2) aufweist:
- eine Abgabeöffnung (20) an einem Ende der Pipette; und
- eine Fahne (3) am gegenüberliegenden Ende der Pipette (2);
- mit einer Abgabekammer (19) zum Aufbewahren einer Flüssigkeit (13) dazwischen, wobei die Abgabekammer (19) zum Abgeben der Flüssigkeit (13) durch die Abgabeöffnung (20) zusammendrückbar ist;
- wobei die Fahne (3) einen plattenförmigen Körper aufweist, wobei der plattenförmige Körper zwei axial-parallele Seitenkanten (50) umfasst;
- wobei die Fahne (3) ein Fahnenende (4) aufweist und das Fahnenende (4) Teil des plattenförmigen Körpers ist;
wobei der Aufbewahrungsbehälter (1) aufweist:
- ein rohrförmiges Gehäuse (5), das an seinem ersten Ende eine erste Öffnung (6) aufweist; und
- zumindest einen ersten Deckel (7) zum entfernbaren Verschließen der ersten Öffnung (6) des rohrförmigen Gehäuses (5), wobei der erste Deckel eine Aufnahme (8) aufweist ausgebildet
- um das Fahnenende (4) an dem ersten Deckel (7) zu befestigen, wobei zumindest die Abgabekammer vollständig aus der Aufnahme (8) herausragt und
- um die Pipette auszurichten, zum Einführen der Pipette (2) in das rohrförmige Gehäuse (5) mit dem einen Ende zuerst, das die Abgabeöffnung (20) aufweist,
wobei der Deckel (7) die erste Öffnung (6) des Aufbewahrungsbehälters (1) verschließt, während die Pipette (2) an dem Deckel (7) befestigt ist,
wobei die Aufnahme (8) eines der folgenden Elemente umfasst
a) eine oder mehrere Federelemente (17);
b) ein Maul (18), das ein oder mehrere unbewegliche Klemmelemente umfasst, die eine Verengung des Mauls (18) bereitstellen, wobei die Verengung eine Verformung oder Verkeilung des Fahnenendes (4) bereitstellt, wenn in das Maul eingeführt;
c) einen Kniehebel oder Schraubklemme;
d) eine Lanze (83) mit einer Lanzenspitze zum Einführen der Spitze in das Fahnenende (4).

2. Der Aufbewahrungsbehälter (1) nach Anspruch 1, wobei der zumindest erste Deckel (7) und die Aufnahme (8) so gestaltet sind, dass die Abgabekammer (19) der Pipette (2) mit den Fingern zusammengedrückt werden kann, um Flüssigkeit aus der Abgabeöffnung (20) der Pipette (2) abzugeben, während das Fahnenende (4) der Pipette (2) in der Aufnahme (8) eingeführt ist.

3. Der Aufbewahrungsbehälter (1) nach Anspruch 1, wobei die Klemmvorrichtung (8) einen Schlitz (18) und ein Federelement (17), einen Kniehebel oder eine Schraubklemme aufweist, wobei das Maul (18) vorzugsweise eine schlitzförmige Öffnung aufweist.

4. Der Aufbewahrungsbehälter (1) nach mindestens einem der vorhergehenden Ansprüche, wobei der Deckel (7) einen Deckelboden (33) aufweist, der Deckelboden (33) eine von einer ringförmigen Schürze umgebene Innenfläche aufweist, die einen Hohlraum bildet, wobei der Hohlraum die Aufnahme (8) umfasst, wobei die Abgabekammer (19) vorzugsweise vollständig aus dem Hohlraum herausragt, wenn das Fahnenende (4) in die Aufnahme (8) eingesetzt ist.

5. Der Aufbewahrungsbehälter (1) nach mindestens einem der vorhergehenden Ansprüche, wobei das rohrförmige Gehäuse (5) an seinem zweiten Ende
a) eine zweite Öffnung (9) aufweist und der Aufbewahrungsbehälter (1) einen zweiten Deckel (10) zum Verschließen und Öffnen der zweiten Öffnung (9) aufweist, oder
b) einen Gehäuseboden (11) aufweist,
wobei der zweite Deckel (10) vorzugsweise auf seiner der zweiten Öffnung (9) des Gehäuses (5) zugewandten Seite eine weitere Aufnahme (8') für das Fahnenende (4) einer weiteren Pipette (2,2`) zum Anbringen der weiteren Pipette (2,2') am zweiten Deckel aufweist.

6. Aufbewahrungsbehälter (1) nach zumindest einem der vorhergehenden Ansprüche, wobei das erste Ende und das zweite Ende des Gehäuses (5) sich entlang der Längsachse (12) des Gehäuses gegenüberliegen.

7. Der Aufbewahrungsbehälter (1) nach zumindest einem der vorhergehenden Ansprüche, wobei der erste Deckel (7) oder der erste Deckel (7) und der zweite Deckel (10) jeweils mit Pipette (2,2`) in der Aufnahme (8,8`) und ohne Pipette in der Aufnahme (8,8`) von dem Gehäuse (5) abnehmbar ist/sind und wobei erste Deckel (7) und/oder der zweite Deckel (10) frei beweglich ohne das Gehäuse ist/sind.

8. Der Aufbewahrungsbehälter (1) nach zumindest einem der vorhergehenden Ansprüche, wobei die Länge des Aufbewahrungsbehälters 3 bis 15 cm und die Breite 1 bis 4 cm beträgt.

9. Der Aufbewahrungsbehälter (1) nach zumindest einem der vorhergehenden Ansprüche, wobei die Aufnahme (8) durch eine oder mehrere Federwangen (17) zum klemmenden Haltern des Fahnenendes (4) ausgebildet ist und die Aufnahme (8) ggf. weiterhin zumindest einen Führungsanschlag (29) und/oder zumindest eine Führungsfläche (39) aufweist.

10. Der Aufbewahrungsbehälter (1) nach zumindest einem der vorhergehenden Ansprüche, wobei die Aufnahme (8) in Form eines Mauls (18) ausgebildet ist, wobei das Fahnenende (4) klemmend in das Maul (18) einschiebbar ist und das Maul (18) vorzugsweise
a) in Einschubrichtung einen wellen- oder S-förmigen Gang (69) hat,
b) einmal bogenförmig gekrümmt ist,
c) mindestens einen Vorsprung (64) in oder an der großflächigen Wand des Mauls aufweist, um eine Einschnürung bereitzustellen,
d) sich zum Ende des Mauls (18) hin klemmend verengt oder in Richtung einer Drehendstellung.

11. Der Aufbewahrungsbehälter (1) nach zumindest einem der vorhergehenden Ansprüche, wobei das Gehäuse (5) und evtl. auch der Deckel (7) doppelwandig und/oder thermisch isolierend ausgebildet ist.

12. Ein Set aufweisend zumindest den Aufbewahrungsbehälter (1) nach zumindest einem der vorhergehenden Ansprüche und zumindest eine Pipette (2), wobei die Pipette (2) aus einem Kunststoffmaterial gefertigt ist, das eine Hauptlängsachse (14) aufweist, entlang der die Pipette (2) aufweist:
- eine Abgabeöffnung (20) an einem Ende der Pipette; und
- eine Fahne (3) am gegenüberliegenden Ende der Pipette (2);
- mit einer Abgabekammer (19) zum Aufbewahren einer Flüssigkeit (13) dazwischen, wobei die Abgabekammer (19) zum Abgeben der Flüssigkeit (13) durch die Abgabeöffnung (20) zusammendrückbar ist;
- wobei die Fahne (3) einen plattenförmigen Körper aufweist, wobei der plattenförmige Körper zwei axial-parallele Seitenkanten (50) umfasst;
- wobei die Fahne (3) ein Fahnenende (4) aufweist und das Fahnenende (4) Teil des plattenförmigen Körpers ist, und
wobei die zumindest eine Pipette (2) an dem ersten Deckel (7) mit dem Fahnenende (4) angebracht ist, wobei das Fahnenende (4) in der Aufnahme (8) eingeführt ist.

13. Das Set nach Anspruch 12, wobei die Fahne (3) einschließlich des Fahnenendes (4) die Form eines länglichen, flachen Quaders hat.

14. Das Set nach zumindest einem der Ansprüche 12 oder 13, wobei der Aufbewahrungsbehälter (1)
a) eine Pipette (2) parallel zur Längsachse (12) des Gehäuses (5) enthält und das Gehäuse (5) mit einem Deckel (7) und einem Gehäuseboden (11) oder zwei Deckeln (7,10) verschlossen ist, wobei zumindest ein Deckel (7) eine Aufnahme (8) zum Einklemmen für das Fahnenende (4) der Pipette (2) aufweist; oder
b) zwei Pipetten (2,2`) hintereinander und entlang der Längsachse (12) des Gehäuses (5) enthält und zwei Deckel (7,10) das Gehäuse (5) verschließen mit jeweils einer Aufnahme (8,8`) zum Einklemmen des Fahnenendes (4) der jeweiligen Pipette (2,2`), wobei die Aufnahmen (8,8`) verschieden sein können; oder
c) zwei Pipetten (2,2`) parallel nebeneinander und parallel zur Längsachse (12) des Gehäuses (5) enthält und das Gehäuse (5) mit einem Deckel (7) und einem Gehäuseboden (11) oder mit zwei Deckeln (7,10) verschlossen ist, wobei der erste Deckel (7) eine Aufnahme für die Fahnenenden (4) von bis zu zwei Pipetten oder zwei Aufnahmen für das Fahnenende (4) von jeweils einer Pipette aufweist und wobei der zweite Deckel (10) keine Aufnahme besitzt.

15. Das Set nach mindestens einem der Ansprüche 12 bis 14, wobei
- der erste Deckel (7) die Öffnung (6) des Aufbewahrungsbehälters (1) verschließt,
- die Längsachse (14) der ersten Pipette (2) im Wesentlichen parallel zur Längsachse (12) des rohrförmigen Gehäuses (5) des Aufbewahrungsbehälters (1) ausgerichtet ist,
- die Pipette (2) im Aufbewahrungsbehälter (1) im Wesentlichen freischwebend angeordnet ist, wobei die Abgabenöffnung (20) von den Innenwänden des Aufbewahrungsbehälters (1) beabstandet ist.

16. Das Set nach zumindest einem der Ansprüche 12 bis 15, wobei die Abgabekammer (19) 0,1 bis 1,5 ml, insbesondere 0,2 bis 0,6 ml, einer Flüssigkeit zum Abgegeben durch die Abgabeöffnung (20) durch Zusammendrücken der Abgabekammer (19) enthält.

17. Das Set nach zumindest einem der Ansprüche 12 bis 16, wobei das der Fahne (3) gegenüberliegende vordere Ende der Pipette (2) von einer Verschlusskappe (21) gebildet wird, die die Abgabekammer (19) verschließt und die Verschlusskappe (21) vorzugsweise im Zusammenhang mit dem ersten Gebrauch entfernt wurde oder nach dem Entfernen wieder aufgesetzt wurde.

18. Das Set nach zumindest einem der Ansprüche 12 bis 17, wobei die Pipette (2, 2') aus einem Kunststoffmaterial gefertigt ist, insbesondere zwei zusammengefügten flächigen Kunststoffmaterialen, in denen zumindest die Abgabekammer (19) in Form von Halbschalen ausgeformt ist und - auch davon unabhängig - die Fahne (3) durch zwei in der ebene parallele flache Abschnitte geformt ist oder durch zwei flache Halbschalen.

## Revendications

1. - Récipient de stockage (1) pour le logement hermétique d'au moins une pipette (2), la pipette (2) étant faite d'une matière plastique ayant un axe longitudinal principal (14) le long duquel la pipette (2) comprend :
- une ouverture de distribution (20) à une extrémité de la pipette ; et
- une languette (3) à l'extrémité opposée de la pipette (2) ;
- avec une chambre de distribution (19) pour le stockage d'un fluide (13) entre celles-ci, la chambre de distribution (19) étant apte à être pressée pour distribuer le fluide (13) à travers l'ouverture de distribution (20) ;
- la languette (3) ayant un corps en forme de plaque, le corps en forme de plaque comprenant deux bords latéraux axialement parallèles (50) ;
- la languette (3) comprenant une extrémité de languette (4), l'extrémité de languette (4) faisant partie du corps en forme de plaque ;
le récipient de stockage (1) comprenant :
- un boîtier tubulaire (5) ayant une première ouverture (6) sur sa première extrémité ; et
- au moins un premier couvercle (7) pour fermer de manière amovible la première ouverture (6) du boîtier tubulaire (5), le premier couvercle comprenant un dispositif de serrage (8) formé
- pour fixer l'extrémité de languette (4) sur le premier couvercle (7) avec au moins la chambre de distribution faisant complètement saillie à partir du dispositif de serrage (8) et
- pour aligner la pipette en vue de l'introduction de la pipette (2) dans le boîtier tubulaire (5) avec l'extrémité comportant l'ouverture de distribution (20) en premier,
le couvercle (7) fermant la première ouverture (6) du récipient de stockage (1) tandis que la pipette (2) est fixée au couvercle (7),
le dispositif de serrage (8) comprenant l'un parmi
a) un ou plusieurs éléments ressorts (17) ;
b) une encoche (18) comprenant un ou plusieurs éléments de serrage inamovibles assurant un rétrécissement de l'encoche (18), le rétrécissement assurant la déformation ou le coincement de l'extrémité de languette (4) lorsqu'elle est introduite dans l'encoche ;
c) un levier à genouillère ou un collier à vis ;
d) une lance (83) avec une pointe de lance en vue de l'introduction de la pointe dans l'extrémité de languette (4).

2. - Récipient de stockage (1) selon la revendication 1, dans lequel l'au moins un premier couvercle (7) et le dispositif de serrage (8) sont configurés de telle sorte que la chambre de distribution (19) de la pipette (2) peut être pressée avec les doigts pour distribuer du fluide hors de l'ouverture de distribution (20) de la pipette (2) tandis que l'extrémité de languette (4) de la pipette (2) est introduite dans le dispositif de serrage (8).

3. - Récipient de stockage (1) selon la revendication 1, dans lequel le dispositif de serrage (8) comprend une encoche (18) et l'un quelconque parmi un élément ressort (17), un levier à genouillère ou un collier à vis, l'encoche (18) ayant, de préférence, une ouverture en forme de fente.

4. - Récipient de stockage (1) selon au moins une des revendications précédentes, dans lequel le couvercle (7) a une base de couvercle (33), la base de couvercle (33) a une surface intérieure entourée d'une jupe annulaire formant une cavité, la cavité comprenant le dispositif de serrage (8), la chambre de distribution (19) faisant, de préférence, complètement saillie à partir de la cavité lorsque l'extrémité de languette (4) est introduite dans le dispositif de serrage (8).

5. - Récipient de stockage (1) selon au moins une des revendications précédentes, dans lequel le boîtier tubulaire (5) comporte, sur sa seconde extrémité :
a) une seconde ouverture (9) et le récipient de stockage (1) a un second couvercle (10) pour fermer et ouvrir la seconde ouverture (9), ou
b) une base de boîtier (11),
le second couvercle (10) ayant, de préférence, sur sa face tournée vers la seconde ouverture (9) du boîtier (5), un autre dispositif de serrage (8') pour l'extrémité de languette (4) d'une autre pipette (2, 2') pour fixer l'autre pipette (2, 2') au second couvercle.

6. - Récipient de stockage (1) selon au moins une des revendications précédentes, dans lequel la première extrémité et la seconde extrémité du boîtier (5) sont à l'opposé l'une de l'autre le long de l'axe longitudinal (12) du boîtier.

7. - Récipient de stockage (1) selon au moins une des revendications précédentes, dans lequel le premier couvercle (7), ou le premier couvercle (7) et le second couvercle (10), est/sont amovible(s) du boîtier (5) avec la pipette (2, 2') fixée au dispositif de serrage (8, 8') ou la pipette retirée du dispositif de serrage (8, 8'), et le premier couvercle (7) et/ou le second couvercle (10) est/sont librement mobile(s) sans le boîtier (5).

8. - Récipient de stockage (1) selon au moins une des revendications précédentes, dans lequel la longueur du récipient de stockage est de 3 à 15 cm, et la largeur est de 1 à 4 cm.

9. - Récipient de stockage (1) selon au moins une des revendications précédentes, dans lequel le dispositif de serrage (8) comprend une ou plusieurs mâchoires à ressort (17) pour maintenir l'extrémité de languette (4) par un serrage, et le dispositif de serrage (8) comprenant en outre, facultativement, au moins une butée de guidage (29) et/ou au moins une surface de guidage (39).

10. - Récipient de stockage (1) selon au moins une des revendications précédentes, dans lequel le dispositif de serrage (8) est agencé sous la forme d'une encoche (18), l'extrémité de languette (4) étant apte à être introduite dans l'encoche (18) d'une manière à serrage, et l'encoche (18) de préférence
a) a un passage ondulé ou en forme de S (69) dans la direction d'introduction,
b) est incurvée une fois en forme d'arc,
c) a au moins une saillie (64) dans ou sur la paroi de grande surface de l'encoche assurant un rétrécissement,
d) se rétrécit vers l'extrémité arrière de l'encoche (18) d'une manière à serrage ou vers une position finale de rotation.

11. - Récipient de stockage (1) selon au moins une des revendications précédentes, dans lequel le boîtier (5) et facultativement le couvercle (7) sont à double paroi et/ou thermiquement isolants.

12. - Ensemble comprenant au moins le récipient de stockage (1) selon au moins une des revendications précédentes et au moins une pipette (2), la pipette (2) étant faite d'une matière plastique ayant un axe longitudinal principal (14) le long duquel la pipette (2) comprend :
- une ouverture de distribution (20) à une extrémité de la pipette ; et
- une languette (3) à l'extrémité opposée de la pipette (2) ;
- avec une chambre de distribution (19) pour le stockage d'un fluide (13) entre celles-ci, la chambre de distribution (19) étant apte à être pressée pour distribuer le fluide (13) à travers l'ouverture de distribution (20) ;
- la languette (3) ayant un corps en forme de plaque, le corps en forme de plaque comprenant deux bords latéraux axialement parallèles (50) ;
- la languette (3) comprenant une extrémité de languette (4), l'extrémité de languette (4) faisant partie du corps en forme de plaque ; et
l'au moins une pipette (2) étant fixée au premier couvercle (7) par l'extrémité de languette (4) introduite dans le dispositif de serrage (8).

13. - Ensemble selon la revendication 12, dans lequel la languette (3), y compris l'extrémité de languette (4), a la forme d'un cuboïde rectangulaire plat.

14. - Ensemble selon au moins une des revendications 12 ou 13, dans lequel le récipient de stockage (1) :
a) contient une pipette (2) parallèle à l'axe longitudinal (12) du boîtier (5), et le boîtier (5) est fermé par le couvercle (7) et par une base de boîtier (11) ou par deux couvercles (7, 10), au moins un couvercle (7) ayant un dispositif de serrage (8) pour serrer l'extrémité de languette (4) de la pipette (2) ; ou
b) contient deux pipettes séquentielles (2, 2') le long de l'axe longitudinal (12) du boîtier (5) et deux couvercles (7, 10) ferment le boîtier (5), chacun ayant un dispositif de serrage (8, 8') pour serrer l'extrémité de languette (4) de la pipette respective (2, 2'), les dispositifs de serrage (8, 8') pouvant être différents ; ou
c) contient deux pipettes (2, 2') parallèles l'une à l'autre et parallèles à l'axe longitudinal (12) du boîtier (5), et le boîtier (5) est fermé par un couvercle (7) et une base de boîtier (11) ou par deux couvercles (7, 10), le premier couvercle (7) ayant un dispositif de serrage pour serrer les extrémités de languette (4) d'un maximum de deux pipettes ou deux dispositifs de serrage pour l'extrémité de languette (4) d'une pipette chacun, et le second couvercle (10) ne comprenant pas de dispositif de serrage.

15. - Ensemble selon au moins une des revendications 12 à 14, dans lequel
- le premier couvercle (7) ferme l'ouverture (6) du récipient de stockage (1),
- l'axe longitudinal (14) de la première pipette (2) est aligné sensiblement parallèlement à l'axe longitudinal (12) du boîtier tubulaire (5) du récipient de stockage (1),
- la pipette (2) est disposée de manière à flotter sensiblement librement dans le récipient de stockage (1) tandis que l'ouverture de distribution (2) est espacée des parois intérieures du récipient de stockage (1).

16. - Ensemble selon au moins une des revendications 12 à 15, dans lequel la chambre de distribution (19) contient de 0,1 à 1,5 ml, en particulier de 0,2 à 0,6 ml, d'un fluide à distribuer à travers l'ouverture de distribution (20) en pressant la chambre de distribution (19).

17. - Ensemble selon au moins une des revendications 12 à 16, dans lequel l'extrémité avant de la pipette (2) à l'opposé de la languette (3) est formée par un capuchon d'étanchéité (21) qui ferme la chambre de distribution (19), le capuchon d'étanchéité (21) étant, de préférence, mis au rebut lors de la première utilisation ou étant remis en place après avoir été retiré.

18. - Ensemble selon au moins une des revendications 12 à 17, dans lequel la pipette (2, 2') est faite d'une matière plastique, notamment deux matières plastiques plates assemblées, dans lequel au moins la chambre de distribution (19) est réalisée sous la forme de deux demi-coques, et - également indépendamment de cela - la languette (3) est formée par deux sections plates à plans parallèles ou par deux demi-coques plates.
